# EUROPEAN PATENT APPLICATION

(11) **EP 1 887 372 A2**
(43) Date of publication of application: **13.02.2008**
(21) Application number: 07015622.9
(22) Date of filing: 08.08.2007
(51) Int. Cl.: G01R 33/28, G01R 33/38

(54) **Seat, a chair including a seat, and a method of using a magnetic resonance imaging system including a seat**

(30) Priority: 08.08.2006 US 500724
(71) Applicant: Philips Medical Systems MR, Inc., Latham NY 12110 (US)
(72) Inventor: Jonas, Philip A., Delmar, NY 12054 (US); Molyneaux, David A., Micanopy, FL 32667 (US); Reis, Chandra T., Altamont, NY 12009 (US)
(74) Representative: Zimmermann, Gerd Heinrich

(57) **Abstract**

A seat (140; 400; 600; 700; 800; 1000; 1200; 1400), a chair (120; 200; 300), or a combination thereof can be part of or used in conjunction with an MRI system (100). The seat can be designed to allow for an MRI analysis without requiring insertion of a probe into a cavity of a patient. In one embodiment, the seat and chair can be adapted for use with a cylindrical-type MRI system. The seat and chair may be used with another type of MRI system, such as an open MRI system. The pelvic region or adjacent portion of a patient can be analyzed without the patient having to lie flat.

## Description

The present disclosure is related to United States Patent Application Number 11/500,724 entitled "Seat, a chair including a seat, and a method of using a magnetic resonance imaging system including a seat" by Jonas et al. filed on August 8, 2006, which is assigned to the current assignee hereof and incorporated herein by reference in its entirety.

The disclosure relates to seats, chairs, and methods, and more particularly to seats, chairs including seats, and methods of using magnetic resonance imaging systems including seats.

Magnetic resonance imaging ("MRI") systems are widely used for analyzing patients. Substantially any region of a patient can be analyzed, including the pelvic region. The pelvic region can be of particular interest when analyzing the prostate, ovaries, colon, another portion of the pelvic region, or any combination thereof. A probe having a radio-frequency ("RF") coil can be inserted into a cavity of the patient while performing the analysis. The procedure is invasive. If the probe is not used, the quality of the image from the region of interest is not as good as compared to when the probe is used and could potentially lead to an improper diagnosis.
In light of the above, a seat for use with a magnetic resonance imaging system according to independent claim 1, the magnetic resonance imaging system, according to claim 3, the chair for use with a magnetic resonance imaging system according to independent claim 4 and a method of using a magnetic resonance imaging system according to independent claim 8 are provided.
Further advantages, features, aspects and details of the invention are evident from the dependent claims, the description and the drawings.

The present disclosure may be better understood, and its numerous features and advantages made apparent to those skilled in the art, by referencing the accompanying drawings.

FIG. 1 includes an illustration of an MRI system, a chair including a seat, and a patient in the chair.

FIG. 2 includes an illustration of a chair illustrating a location to receive a seat.

FIG. 3 includes an illustration of another chair illustrating another location to receive a seat.

FIGs. 4 and 5 include illustrations of a top view and a cross-sectional view, respectively, of a seat designed for a female patient.

FIGs. 6 and 7 include illustrations of top views of the seat of FIG. 4 and locations where sensors may be located within the seat.

FIGs. 8 and 9 include illustrations of a top view and a cross-sectional view, respectively, of a seat designed for a male patient.

FIGs. 10 and 11 include illustrations of a top view and a cross-sectional view, respectively, of a seat with a flexible protruding portion.

FIGs. 12 and 13 include illustrations of a top view and a cross-sectional view, respectively, of a seat designed for a lower back of a patient.

FIG. 14 includes an illustration of a cross-sectional view of a seat including openings and a probe.

FIG. 15 includes a flow diagram of a method of using an MRI system.

The use of the same reference symbols in different drawings indicates similar or identical items. Skilled artisans will appreciate that elements in the figures are illustrated for simplicity and clarity and have not necessarily been drawn to scale.

A seat, a chair, or a combination thereof can be part of or used with in conjunction with an MRI system. The seat can be designed to allow for an MRI analysis without requiring insertion of a probe into a cavity of a patient. In one embodiment, the seat and chair can be adapted for use with a cylindrical-type MRI system. The seat and chair may be used with another type of MRI system, such as an open MRI system. The pelvic region or adjacent portion of a patient can be analyzed without the patient having to lie flat or sandwiched between two large primary magnets. The designs and methods herein can help make the patient's MRI experience more enjoyable by allowing a patient to sit, not requiring most of the patient to be inserted into an opening in the MRI system (reduce the likelihood of a claustrophobic feeling), not requiring an invasive probe, or any combination thereof.

In a first aspect, a seat for use with a magnetic resonance imaging system can include a first protruding portion and a first sensor disposed within the first protruding portion. The first sensor can sense when the magnetic resonance imaging system would be used. In a second aspect, a chair for use with a magnetic resonance imaging system can include a first section configured to support a back of a patient, a second section configured to receive a first seat that includes a first sensor, wherein the first sensor is capable of providing data for use in an imaging process, and a bend adjacent to the first section and the second section. In a third aspect, a method of using a magnetic resonance imaging system can include disposing a first patient within the magnetic resonance imaging system, and analyzing the first patient using the magnetic resonance imaging system. Analyzing can be performed when the first patient is adjacent to a first seat that includes a first sensor, and analyzing can be performed using the first sensor.

A few terms are defined or clarified to aid in understanding of the terms as used throughout this specification.

The term "analyzing region," with respect to an MRI system, is intended to mean a region where a patient or other object can be properly analyzed when using the MRI system.

The term "chair" is intended to mean a structure or other object used to support the back and legs of patient when the patient would be in a sitting position.

The term "gradient coil" is intended to mean a coil or a set of coils that can be used during analysis of a patient to affect a magnetic field generated by a primary magnet. Typically, as is common in the art, "gradient coil" refers to any system that creates a time-varying modulation of the primary magnetic field for a purpose of focusing data collection on a particular location.

The term "pass-through object" is intended to mean tubing, a sensor, a probe, a scalpel, a syringe, a biopsy needle, or other instrument used in analyzing, diagnosing, treating, or performing a medical procedure on a patient, wherein at least a portion of such tubing, sensor, probe, scalpel, syringe, biopsy needle, or other instrument is capable of being passed or extending through an opening in a magnetic resonance imaging system while a primary magnet of the system is at a magnetic field substantially greater than 0.0 T.

The term "pelvic region" is intended to mean a region of a patient that extends from a plane substantially perpendicular to length of the patient and including the top of a hip bone (iliac crest of the ilium) to another plane substantially perpendicular to length of the patient and including the bottom of a bone adjacent to the public arch (ischium).

The term "primary magnet" is intended to mean a magnet capable of generating a magnetic field that is significantly stronger than a gradient coil.

The term "seat" is intended to mean a structure or other object used to support the buttocks of a patient, the pelvic region of a patient, or a combination thereof when the patient would be in a sitting position.

The term "typical operating state" is intended to mean a state in which all superconducting elements along a superconducting current path are in their superconducting states.

As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

Additionally, for clarity purposes and to give a general sense of the scope of the embodiments described herein, the use of the "a" or "an" are employed to describe one or more articles to which "a" or "an" refers. Therefore, the description should be read to include one or at least one whenever "a" or "an" is used, and the singular also includes the plural unless it is clear that the contrary is meant otherwise.

Unless otherwise defined, all technical and scientific terms used herein have the same meaning as commonly understood by one of ordinary skill in the art to which this invention belongs. All publications, patent applications, patents, and other references mentioned herein are incorporated by reference in their entirety. In case of conflict, the present specification, including definitions, will control. In addition, the materials, methods, and examples are illustrative only and not intended to be limiting.

To the extent not described herein, many details regarding specific materials, processing acts, and components, assemblies, and systems are conventional and may be found in textbooks and other sources within the superconducting, cryogenic, and medical device arts.

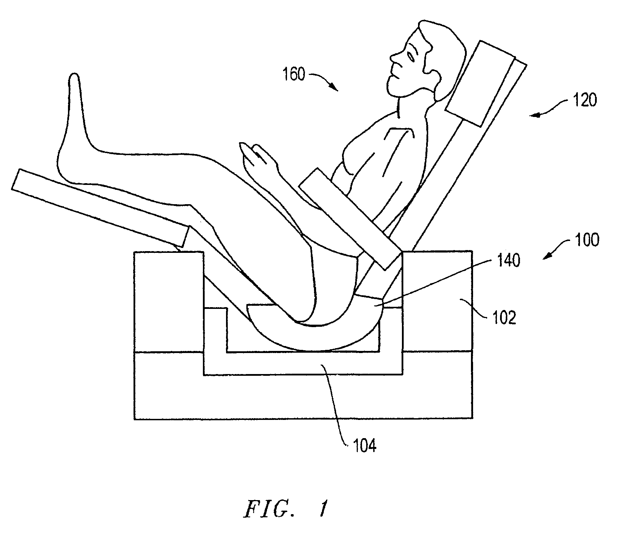

FIG. 1 includes an illustration of an MRI system 100 that includes a primary magnet section 102 and a gradient coil section 104. The primary magnet section 102 can include a primary magnet in the form of a solenoid that may include a superconducting wire. In one embodiment, the primary magnet section 102 can include one primary magnet, and in another embodiment can include more than one primary magnet. The primary magnet section 102 can generate a relatively large, substantially static magnetic field when the MRI system 100 is in its typical operating state. In one embodiment, the magnetic field can be at least 0.1, 0.5, or 0.9 T, and in another embodiment, the magnetic field may not exceed 10, 5, or 3 T. In still other embodiments, a stronger or weaker magnetic field may be used with the primary magnet.

The gradient coil section 104 includes a gradient coil. The gradient coil can be a cylindrical style, pancake style, or a combination that includes a cylindrical portion and a substantially flat portion. In a particular embodiment where the gradient coil is the pancake style or has the substantially flat portion, the gradient coil may or may not include an opening through which an object (e.g., a probe, a tube, etc.) can pass. In one embodiment, the gradient coil section 104 can include one gradient coil, and in another embodiment can include more than one gradient coil (e.g., a set of coils).

The MRI system 100 can include another section not illustrated. For example, the MRI system 100 can include a vessel that contains a cryogenic liquid, electrical and electronic sub-systems for operating the MRI system 100, magnetic field shielding (active, passive, or a combination thereof), another suitable sub-system, or any combination thereof.

FIG. 1 also includes a chair 120, a seat 140, and patient 160 disposed within the MRI system 100. The chair 120 and seat 140 may or may not be part of the MRI system 100. Designs and other configuration options of chairs and seats are described in more detail below.

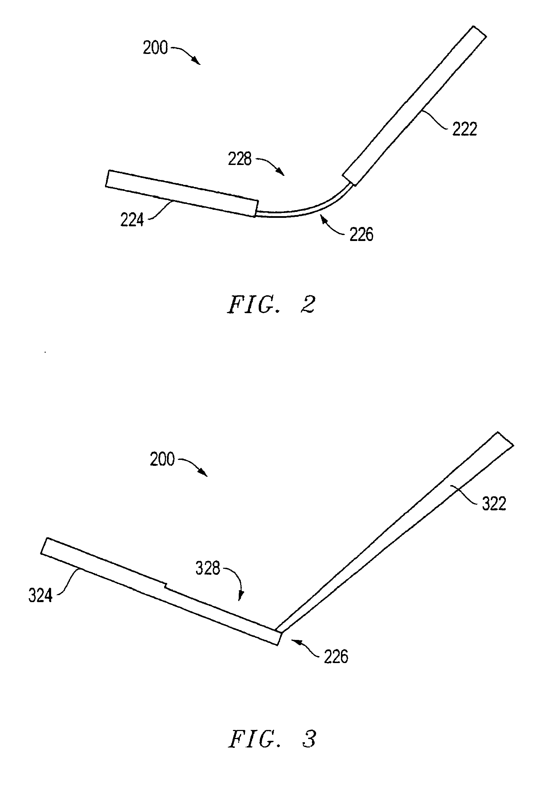

FIGs. 2 and 3 include illustrations of side views of chairs that can be used with the MRI system 100. In FIG. 2, the chair 200 can include a first section 222 having a portion that is capable of supporting the back of a patient, a second section 224 having a portion that is capable of supporting the legs of the patient, and a transition section 226 disposed between the first section 222 and the second section 224. The transition section 226 can form a substantially continuous bend.

In one embodiment, the transition section 226 can include a substantially solid portion that is relatively thinner than the first section 222, the second section 224, or both. In another embodiment, the transition section 226 can include rods, tubes, other frame members, or any combination thereof, wherein such rods, tubes, other frame members, or any combination thereof extend into the first section 222, the second section 224, or both. In a particular embodiment, the transition section 226 may include a pair of spaced-apart rods or tubes. The space between the rods or tubes can allow a pass-through object to pass or extend through the space. After reading this specification, skilled artisans will appreciate that other configurations are possible and still form a bend that is substantially continuous.

A region 228 is an open region adjacent to the transition section 226 where a seat (not illustrated in FIG. 2) may be placed. The seat may merely rest along the inner part of the transition section 226 or may be attached to the chair 200. Seat designs and configurations are discussed in more detail later in this specification. In FIG. 2, the region 228 is bounded on three of its sides by the first section 222, the second section 224, and the transition section 226.

In FIG. 3, the chair 300 can include a first section 322 having a portion that is capable of supporting the back of a patient and a second section 324 having a portion that is capable of supporting the legs of the patient. In this particular embodiment, a bend 326 is discontinuous and is where the first section 322 abuts the second section 324.

A region 328 can be an open region adjacent that is a recession within another portion of the second section 324, wherein the other portion is adjacent to the bend 326. The region 328 can be where a seat (not illustrated in FIG. 3) may be placed. Similar to the region 228 in FIG. 2, the seat may merely rest within the region 328 or may be attached to the chair 300. In another embodiment, the other portion of the second section 324 (underneath the region 328) can include rods, tubes, other frame members, or any combination thereof. In a particular embodiment, the other portion of the first section 322 may include a pair of spaced-apart rods or tubes. The space between the rods or tubes can allow a pass-through object to pass or extend through the space. The section 322 may include a portion (not illustrated) that is capable of receiving part of a seat. After reading this specification, skilled artisans will appreciate that other configurations for chair 300 are possible.

Different design or configuration features of chairs 200 and 300 may be combined to make other chairs. Still other features (not illustrated) may be used. For example, a chair may include lumbar support, an armrest, a pillow or other headrest, heating or cooling elements, or any combination thereof. The bend defined by the transition section 226 or the bend 326 may be adjustable. The second section 224 or 324 may allow for another bend at a location that would be near where a knee of the patient would be located when present in the chair. After reading this specification, skilled artisans will appreciate that the chair may include other features.

The chair may be substantially permanently attached to the MRI system 100 or may be removable with respect to the MRI system 100. If substantially permanently attached, the chair may be attached using a weld, a screw, a bolt, a bolt and nut, a rivet, another suitable mechanical fastener, or any combination thereof. A patient would be disposed in the chair while a portion of the chair is disposed within an analyzing region of the MRI system 100. If the chair is removable, the patient may be disposed in the chair before or after the chair is removed from the analyzing region of the MRI system 100. If needed or desired, a relatively temporary fastener or alignment structure may be used to reduce the likelihood that the chair will move when a patient is being analyzed using the MRI system 100. Such relatively temporary fastener or alignment structure can include a latch, a hole-and-pin assembly, complementary features (male and female features), another suitable relatively temporary fastener or alignment structure, or any combination thereof. The manner in which the chair is secured into position within the MRI system 100 can be varied to meet the needs or desires of the MRI system designer or operator.

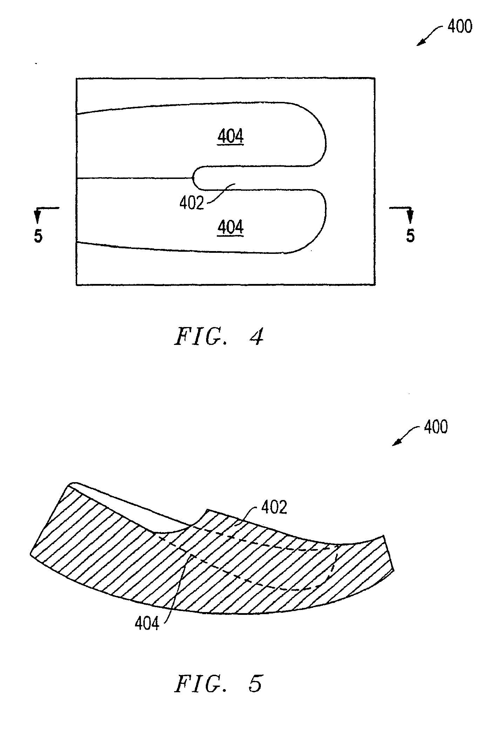

FIGs. 4 through 14 illustrate different seats that can be used with the chair 200. The seats can be modified to fit chair 300 or other designs of chairs. The different seats can be used for different patients or for analyzing different areas of the same patient. Many of the designs for the seats are similar to a bicycle seat design in that the patient may be supported more by bones of the pelvis, as opposed to buttocks or legs of the patient. Other designs for the seats can allow for more support of the buttocks, legs, or any combination thereof, and therefore, support by bones of the pelvis is not required. The seats can include a protruding portion configured to separate buttocks near an anus of the patient, legs of the patient, flesh near a vaginal opening of the patient, or any combination thereof. The seats can allow for better analysis of the prostate, uterus, ovaries, and other parts of the body within or adjacent to the pelvic region of the patient. Other protruding portions can be used to analyze an anterior portion or a posterior portion of the patient. After reading this specification, skilled artisans will appreciate that many other designs are possible.

FIG. 4 includes an illustration of a top view of a seat 400 that can be used with a female patient. FIG. 5 includes an illustration of a cross-sectional view at sectioning line 5-5 in FIG. 4. The seat 400 can include a protruding portion 402 and recessed portions 404. The protruding portion 402 can help to separate flesh adjacent to the vaginal and anal openings of the patient. The recessed portions 404 can receive legs, buttocks, or combination thereof of the patient. The recessed portions 404 are not required.

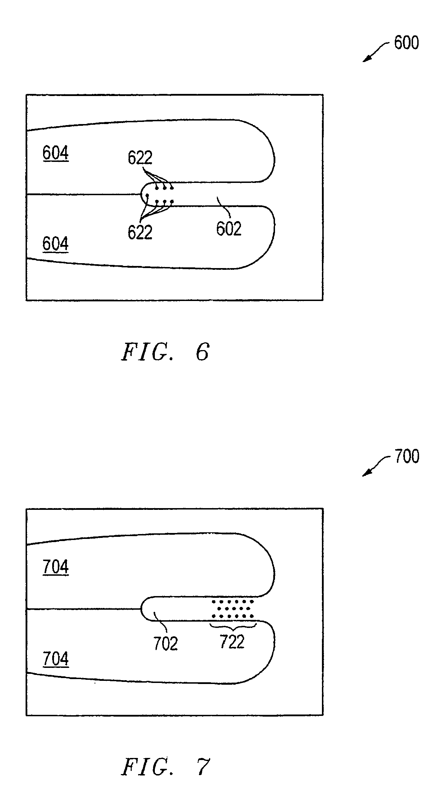

FIGs. 6 and 7 include illustrations of top views of seats that are similar to the seat 400. In FIG. 6, a seat 600 includes a protruding portion 602 and recessed portions 604, similar to the protruding sections 402 and recessed portions 404 of the seat 400. The protruding section 602 can include sensors 622 used in analyzing. The sensors 622 can include an RF coil, an inductor, or other conductive loop. The RF coil can be part of a receiver, a transmitter, or a transceiver. The sensors 622 can be coupled to other electronics (not illustrated) of the MRI system 100. The location of sensors 622 is well suited for analysis of the uterus, ovaries, and fallopian tubes of the patient. In FIG. 7, a seat 700 includes a protruding portion 702 and recessed portions 704, similar to the protruding sections 402 and recessed portions 404 of the seat 400. The protruding section 702 can include sensors 722 used in analyzing may be located. The sensors 722 can be coupled to other electronics (not illustrated) of the MRI system 100. The location of sensors 722 is well suited for analysis of the colon of the patient.

In another embodiment, more or fewer sensors 622 or 722 may be used. In still another embodiment, any combination of sensors 622 and 722 may be used in the same seat. If different areas are more of a focus for analysis, different sets of sensors 622 or 722 may be active or data from some but not all of the sensors 622 or 722 may be used. Thus, the same seat or different seats may be used to analyze different areas of the same patient.

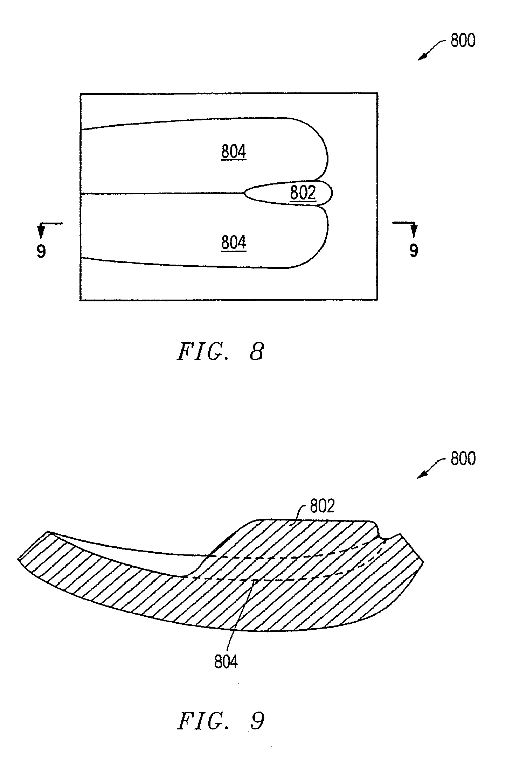

FIG. 8 includes an illustration of a top view of a seat 800 that can be used with a male patient. FIG. 9 includes an illustration of a cross-sectional view at sectioning line 9-9 in FIG. 8. The seat 800 can include a protruding portion 802 and recessed portions 804. The protruding portion 802 can help to separate flesh adjacent to the anal openings of the patient. As compared to the protruding portion 402 in FIG. 4, the protruding portion 802 in FIG. 8 has a shorter length. The recessed portions 804 can receive legs, buttocks, or combination thereof of the patient. The recessed portions 804 are not required.

The protruding portion 802 can include sensors, similar to the sensors described with respect to FIGs. 6 and 7. The seat 800, and particularly the protruding portion 802 may include sensors at different positions, and one, some, or all of the sensors may be used during analysis of the patient. Although not illustrated, other seat shapes may be used. In one particular embodiment, a seat may have smaller features to accommodate patients that are children. In another embodiment, another seat may be used for patients that are significantly larger than the average size of an adult patient.

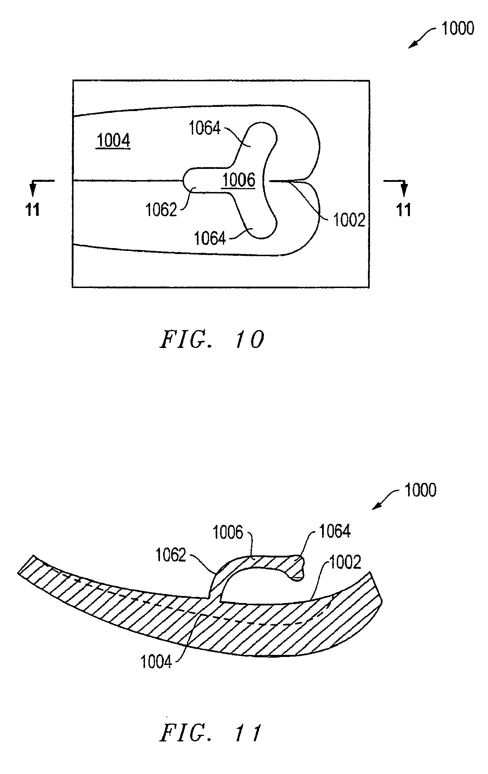

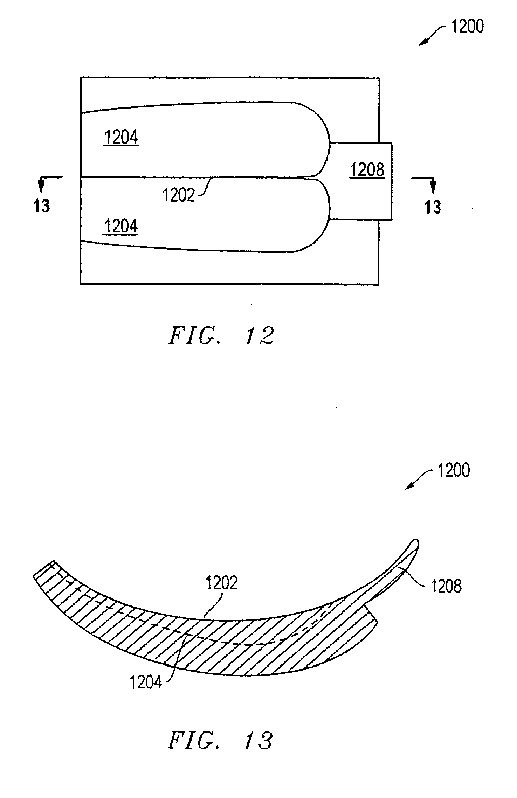

FIG. 10 includes an illustration of a top view of a seat 1000 that can be used when analyzing an anterior portion a patient. FIG. 11 includes an illustration of a cross-sectional view at sectioning line 11-11 in FIG. 10. The seat 1000 can include a protruding portion 1002 and recessed portions 1004. The recessed portions 1004 can receive legs, buttocks, or combination thereof of the patient. The recessed portions 1004 are not required.

The seat 1000 can also include another protruding portion 1006 that wraps around to the front of the patient. The protruding portion 1006 can include a stem 1062 that would extend between the legs of the patient when being analyzed and extensions 1064 that extend laterally from the stem 1062. The protruding portion 1006 may be permanently or temporarily attached to the seat 1000. The protruding portion 1006 can be rigid or may be flexible to allow the protruding portion 1006 to be positioned for a particular patient. Other designs (not illustrated) for the protruding portion 1006 may not include the extensions 1064, have larger or smaller extensions 1064, or have an extension that extends around the sides and back of the patient. In still another design, the stem 1062 is not present, in which case, the protruding portion is similar to a strap across the patient that may or may not have a varying width. Such a protruding portion can be attached at another location, other than between the legs of the patient. After reading this specification, skilled artisans will appreciate that other designs can be used.

The protruding portion 1002, the protruding portion 1006, or both can include sensors, similar to the sensors described with respect to FIGs. 6 and 7. With respect to the protruding portion 1006, sensors may be located in the stem 1062, one or both of the extensions 1064, or any combination thereof. In a particular embodiment, the protruding portion 1006 can be adjacent to a testicle of the patient, a penis of the patient, a pelvic region of the patient, skin adjacent to the pelvic region, or any combination thereof. The pelvic region can include a bone of a pelvis of a patent, an organ disposed between any bones within the pelvis of the patient (e.g., ovaries, fallopian tubes, large intestines, small intestines, colon, bladder, prostate, etc.), or any combination thereof. The sensors can be located within the protruding portion 1006 when analyzing any of the foregoing or another part of the patient. One, some, or all of the sensors in seat 1000 may be used during analysis of the patient.

FIG. 12 includes an illustration of a top view of a seat 1200 that can be used when analyzing a posterior portion a patient. FIG. 13 includes an illustration of a cross-sectional view at sectioning line 13-13 in FIG. 12. The seat 1200 can include a protruding portion 1202 and recessed portions 1204. The recessed portions 1204 can receive legs, buttocks, or combination thereof of the patient. The recessed portions 1204 are not required.

The seat 1200 can also include another protruding portion 1208 that extends along a back of the patient. Similar to the protruding portion 1006 in FIG. 10, the protruding portion 1208 may be permanently or temporarily attached to the seat 1200. The protruding portion 1208 can be rigid or may be flexible to allow the protruding portion 1208 to be positioned for a particular patient. Other designs (not illustrated) for the protruding portion 1208 may extend further or not as far along the back of the patient. In still another design (not illustrated), the protruding portion 1208 may partly or completely wrap around the torso of the patient. After reading this specification, skilled artisans will appreciate that other designs can be used.

The protruding portion 1202, the protruding portion 1208, or both can include sensors, similar to the sensors described with respect to FIGs. 6 and 7. In a particular embodiment, the protruding portion 1208 can be adjacent to a pelvic region of the patient, skin adjacent to the pelvic region, spine of the patient (e.g., lumbar vertebrae), or any combination thereof. The sensors can be located within the protruding portion 1208 when analyzing any of the foregoing or another part of the patient. One, some, or all of the sensors in seat 1000 may be used during analysis of the patient.

In still another embodiment, a combination of the foregoing designs can be used. The protruding portions 402, 602, 702, 802, 1002, 1202, 1006, and 1208 can be mixed and matched, as needed or desired. For example, the reproductive organs of a female patient may be the regions of particular interest when analyzing a female patient. The seat may include protruding portions 402 and 1006. The uterus may be analyzed using sensors in the protruding portion 402 and stem 1062 of the protruding portion, the fallopian tubes may be analyzed using sensors in the stem 1062 and extensions 1064 of the protruding portion 1006, and the ovaries may be analyzed using sensors in the extensions 1064 of the protruding portion 1006. In another example, the colon of a patient may be the region of particular interest when analyzing the patient. The seat may include protruding portions 402 and 1208 for a female patient or protruding portions 802 and 1208 for a male patient. Sensors would be placed at appropriate locations within the protruding portions to analyze the colon of the patient.

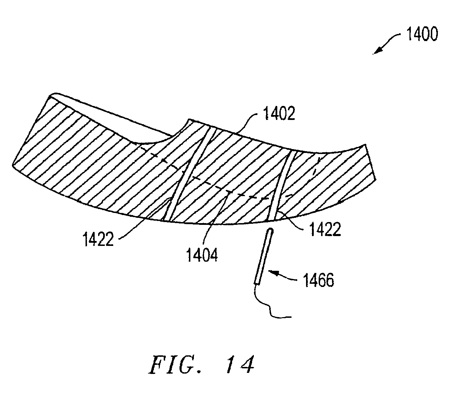

The designs of the seat, including locations of the protruding portions and locations of sensors, can allow for analysis of a patient using an MRI system without the need of a probe. Still, in another embodiment, a probe can be used with a seat, if desired. FIG. 14 includes an illustration of a cross-sectional view of a seat 1400 that can be used with a probe 1466 that includes a sensor. The seat 1400 can include a protruding portion 1402 and recessed portions 1404. The recessed portions 1404 are not required.

The seat 1400 can also include an opening 1422, an opening 1424, another suitable opening, or any combination thereof. The probe 1466 can be inserted through the opening 1422, the opening 1424, or another opening in the seat and into a cavity of the patient. In another embodiment, the probe may be inserted into the cavity of the patient before the patient is seated. A wire or another part of the probe 1466 may be fed into the opening of the seat during or after the patient is disposed on the seat.

In still another embodiment, a chair is not needed. A seat may be attached to the MRI system 100 without the chair. Any of the seats previously described or modified versions of those seats could be used. In yet a further embodiment (not illustrated), any of the foregoing seat or chair designs may include or be used in conjunction with a restraint to reduce patient motion during imaging. For any of the foregoing seat or chair designs, a sensor may be partly or completely disposed within a protuding portion or recessed portion of the seat or chair.

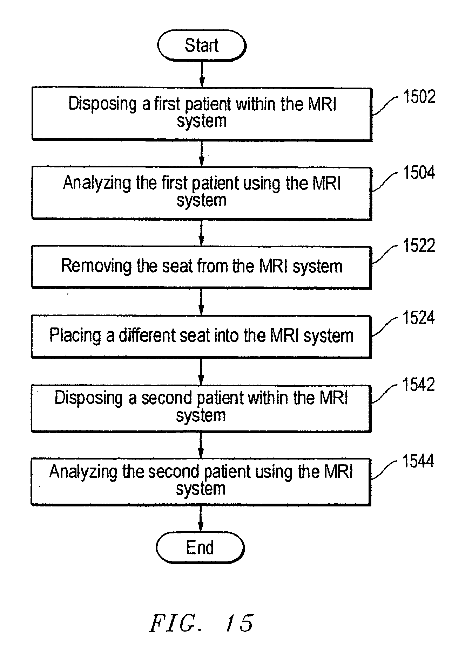

Attention is now directed to methods of using the MRI system 100. Any of the previously described embodiments including a seat and an optional chair may be used. The method can include disposing a first patient within the MRI system, at block 1502 in FIG. 15. In one embodiment, the chair and seat may already be positioned within the MRI system. In a particular embodiment, the first patient may sit in the chair without any assistance, and in another embodiment, the first patient may be assisted. Such assistance may be provided mechanically or by a human. In another embodiment, a human may assist the patient into the chair. In still another embodiment, the first patient may be on the seat and the combination of the first patient and the seat can be lowered into the opening of the MRI system 100. Thus, disposing should be construed broadly and may be performed by the first patient without any assistance or may include assistance.

In any one or more of the embodiments, the method can optionally include positioning the first patient onto the seat. The positioning may be performed before disposing a first patient within the MRI system, during disposing a first patient within the MRI system, after disposing a first patient within the MRI system, or any combination thereof. The position may be performed by the first patient with or without assistance. Positioning may be performed to place the first patient in proper alignment with the sensors, improve comfort of the first patient, or another reason.

The method can also include analyzing the first patient using the MRI system, at block 1504. Analyzing can be performed when the first patient is adjacent to the seat that includes a sensor. The analysis can be performed using the sensor. In one embodiment, the seat includes one sensor, and in another embodiment, the seat includes a plurality of sensors. The sensors may be RF coils or another type of sensor, such as a temperature sensor, a pressure sensor, etc. During analysis, the magnetic field generated by the primary magnet section can be relatively static while the magnetic field generated by the gradient coil section is changed.

During analysis, the first patient may be in a sitting position. In a particular embodiment, a head and a leg of the first patient extend outside of an analyzing region of the MRI system. A first line can be defined to include a first point corresponding to a center of the pelvic region of the first patient and a second point corresponding to a center of the torso at the base of the neck of the patient. A second line can be defined to include the first point and a third point corresponding to a center of a knee of the patient. An angle is formed at an intersection of the first line and the second line may be no greater than 150° in one embodiment, no greater than 120° in another embodiment, and no greater than 100° in still another embodiment. The angle may be at least 60° in one embodiment and at least 80° in another embodiment. In another embodiment, a different angle may be used that is less than 60°, greater than 150°, or has another value between 60° and 150°. In yet another embodiment, the angle can be changed, such that during a first portion of the analysis, one angle is used, and during a second portion of analysis, a different angle is used. After reading this specification, skilled artisans will be able to select an angle that meets their needs or desires.

The method can optionally include inserting a probe into a cavity of the first patient. The probe may be inserted before the first patient is on the seat or while the first patient is on the seat. The probe can include a sensor and be used during the analysis.

The method can further include removing the seat from the MRI system, at block 1522, and placing a different seat into the MRI system, at block 1524. In a particular embodiment, the prior seat can be removed after analyzing the first patient and before analyzing a second patient, and the different seat can be placed into the MRI system before analyzing the second patient. The seats may differ in shape, placement of sensors, in another configuration aspect, or the like. The first patient and the second patient may be the same patient or different patients. If different patients, one patient may be male and the other female, or one patient may be an adult and the other a child. Other combinations of different patients are possible. The different seats may be configured to analyze for different portions of the same patient or different patients. In another particular embodiment, a chair could be used, and the seat change may include removing the prior seat from the chair and placing the different seat on the chair.

The method can still further include disposing a second patient within the MRI system, at block 1542, and analyzing the second patient using the MRI system, at block 1544. The disposing and analyzing of the second patient may be performed using any one or more of the embodiments previously described with respect to the first patient. The actual embodiments used in disposing and analysis of the second patient may be the same or different from those used for the first patient. Additionally, optional act(s), such as positioning the second patient on the different seat, inserting a probe, etc. may also be performed.

After reading this specification, skilled artisans will appreciate that use of the different seats may allow for a better MRI analysis of the pelvic region and adjacent regions of the patient, potentially without the need of a probe. Thus, analysis of reproductive organs, colon, or other part of the patient may be performed without requiring the invasive procedure using a probe. Still, a probe could be used if needed or desired.

The ability to use different seats is beneficial to allow better analysis of different portions of the same patient, the same or different portions of different patients, or the like. Seats may be exchanged relatively easily. If a chair is used, the chair may be useful in providing comfort to patients during analysis, without making them feel trapped as is widespread if a conventional solenoid primary magnet was used. Still further, the chair may be adapted to move is a fashion similar to a lift chair, so that moving-impaired patients (e.g., handicapped or elderly) can be more readily disposed within the MRI system.

Many different aspects and embodiments are possible. Some of those aspects and embodiments are described below. After reading this specification, skilled artisans will appreciate that those aspects and embodiments are only illustrative and do not limit the scope of the present invention.

In a first aspect, a seat for use with a magnetic resonance imaging system can include a first protruding portion and a first sensor disposed within the first protruding portion. The first sensor can provide data used in the imaging process.

In one embodiment of the first aspect, the first protruding section is configured to receive a patient. In a particular embodiment, the first protruding portion is configured to separate buttocks near an anus of the patient, separate legs of the patient, separate flesh near a vaginal opening of the patient, or any combination thereof. In another particular embodiment, the seat further includes a recessed portion configured to receive a buttock or a leg of the patient.

In another embodiment of the first aspect, the first protruding portion is configured to extend to an anterior portion of a patient or a posterior portion of a patient. In a particular embodiment, the first protruding portion is flexible and is capable of being positioned adjacent to a region of interest of the patient. In another particular embodiment, the first protruding portion is adjacent to a testicle of the patient, a penis of the patient, a pelvic region of the patient, skin adjacent to the pelvic region, or any combination thereof. In a more particular embodiment, the pelvic region of the patient includes a bone of a pelvis of the patient, an organ disposed between any bones of the pelvis of the patient, or any combination thereof. In still another particular embodiment of the first aspect, the seat further includes a second protruding portion including a second sensor, wherein the second sensor is capable of providing data for use in an imaging process. The second protruding portion is configured to separate buttocks near an anus of the patient, separate legs of the patient, separate flesh near a vaginal opening of the patient, or any combination thereof.

In a second aspect, a chair for use with a magnetic resonance imaging system can include a first section configured to support a back of a patient, a second section configured to receive a first seat that includes a first sensor, wherein the first sensor is capable of providing data for use in an imaging process, and a bend adjacent to the first section and the second section.

In one embodiment of the second aspect, the bend includes a first portion of the first section and a second portion of the second section. In another embodiment, the bend is where the first portion abuts the second portion. In still another embodiment, the second section is also configured to receive a second seat that includes a second sensor. The second seat can have a different design as compared to the first seat, and the second sensor provide data used in the imaging process. In a particular embodiment, the first seat is designed primarily for a male patient as compared to a female patient, and the second seat is designed primarily for the female patient as compared to the male patient. In another particular embodiment, the first seat is designed primarily for an adult patient as compared to a child patient, and the second seat is designed primarily for the child patient as compared to the adult patient. In still another particular embodiment, the first seat is designed primarily to analyze a first portion of the patient, the second seat is designed primarily to analyze a second portion of the patient, the first portion of the patient includes a first organ and not a second organ, and the second portion of the patient includes the second organ and not the first portion.

In still another embodiment, a magnetic resonance imaging system can include or use any seat, chair, or combination thereof.

In a third aspect, a method of using a magnetic resonance imaging system can include disposing a first patient within the magnetic resonance imaging system, and analyzing the first patient using the magnetic resonance imaging system. Analyzing can be performed when the first patient adjacent to a first seat that includes a first sensor, and analyzing can be performed using the first sensor.

In one embodiment of the third aspect, the method further includes disposing a second patient within the magnetic resonance imaging system, and analyzing the second patient using the magnetic resonance imaging system. Analyzing can be performed when the second patient adjacent to a second seat that includes a second sensor, and analyzing is performed using the second sensor. The second seat can have a different design as compared to the first seat. In a particular embodiment, the method further includes removing the first seat from the magnetic resonance imaging system after analyzing the first patient and before analyzing the second patient, and placing the second seat into the magnetic resonance imaging system before analyzing the second patient. In another particular embodiment, the first patient is a male patient, and the second patient is a female patient. In still another particular embodiment, the first patient is an adult patient, and the second patient is a child patient. In yet another particular embodiment, the first patient and the second patient are a same patient, analyzing the first patient comprises analyzing a first portion of the same patient, and analyzing the second patient comprises analyzing a second portion of same patient.

In another embodiment of the third aspect, the method further includes inserting a probe into a cavity of the first patient, wherein analyzing the first patient comprises analyzing the first patient using the probe. In still another embodiment, disposing the first patient comprises lowering the first patient into an opening within the magnetic resonance imaging system.
According to yet further embodiments, a seat for use with a magnetic resonance imaging system comprises a first protruding portion, and a first sensor disposed within the first protruding portion, wherein the first sensor is capable of providing data for use in an imaging process. Typically, as a first option, the first protruding section can be configured to receive a patient. Thereby, further optionally, the first protruding portion can be configured to separate buttocks near an anus of the patient, separate legs of the patient, and/or separate flesh near a vaginal opening of the patient. According to an even further alternative or additional embodiment, the seat may further include a recessed portion configured to receive a buttock or a leg of the patient. According to yet further embodiments, which can be combined with other embodiments described herein, the first protruding portion is configured to extend to an anterior portion of a patient or a posterior portion of a patient. Thereby, as an additional typical option the first protruding portion can be flexible and can be capable of being positioned adjacent to a region of interest of the patient. Alternatively or additionally, the first protruding portion can be adjacent to, a testicle of the patient, a penis of the patient, a pelvic region of the patient, and/or skin adjacent to the pelvic region. According to yet further aspects that can be combined with embodiments described herein the pelvic region of the patient can include a bone of a pelvis of the patient, and/or an organ disposed between any bones of the pelvis of the patient. Additionally or alternatively, the seat can typically include a second protruding portion including a second sensor, wherein the second sensor can be capable of providing data for use in an imaging process, and the second protruding portion can be configured to, separate buttocks near an anus of the patient, separate legs of the patient, and/or separate flesh near a vaginal opening of the patient.
According to yet further embodiments, a magnetic resonance imaging system includes a seat of any of the embodiments or combinations described above and an analyzing region, wherein the seat is disposed within the analyzing region.
According to even further embodiments, a chair for use with a magnetic resonance imaging system can include a first section configured to support a back of a patient, a second section configured to receive a first seat that includes a first sensor, wherein the first sensor is capable of providing data for use in an imaging process, and a bend adjacent to the first section and the second section. Thereby, typical embodiments can additionally or alternatively have the bend including a first portion of the first section and a second portion of the second section, and/or the bend can be where the first portion abuts the second portion. According to other embodiments, which ban be arbitrarily combined with other embodiments, the second section can also be configured to receive a second seat that includes a second sensor, wherein the second seat has a different design as compared to the first seat, and the second sensor is capable of providing data for use in an imaging process. Thereby, optionally, the first seat can be designed primarily for a male patient as compared to a female patient, and the second seat is designed primarily for the female patient as compared to the male patient; the first seat can be designed primarily for an adult patient as compared to a child patient, and the second seat is designed primarily for the child patient as compared to the adult patient; or the first seat can be designed primarily to analyze a first portion of the patient, the second seat is designed primarily to analyze a second portion of the patient, the first portion of the patient includes a first organ and not a second organ, and the second portion of the patient includes the second organ and not the first organ.
According to further embodiments, a magnetic resonance imaging system a chair of any of the embodiments or combinations described above and an analyzing region, wherein the bend of the chair can be disposed within the analyzing region.
According to other embodiments, a method of using a magnetic resonance imaging system can include disposing a first patient within the magnetic resonance imaging system, and analyzing the first patient using the magnetic resonance imaging system, wherein analyzing is performed when the first patient adjacent to a first seat that includes a first sensor, and analyzing is performed using the first sensor. Additionally, a method may include disposing a second patient within the magnetic resonance imaging system, and analyzing the second patient using the magnetic resonance imaging system, wherein, analyzing is performed when the second patient adjacent to a second seat that includes a second sensor, analyzing is performed using the second sensor, and the second seat has a different design as compared to the first seat. As a further additional option, a method may include removing the first seat from the magnetic resonance imaging system after analyzing the first patient and before analyzing the second patient; and placing the second seat into the magnetic resonance imaging system before analyzing the second patient. According to different embodiments, which can be combined with other embodiments described herein, the first patient can be a male patient, and the second patient can be a female patient; the first patient can be an adult patient, and the second patient can be a child patient; and/or the first patient and the second patient are a same patient wherein analyzing the first patient can include analyzing a first portion of the same patient, and analyzing the second patient can include analyzing a second portion of same patient. According to even further embodiments, additionally or alternatively, a method can include inserting a probe into a cavity of the first patient, wherein analyzing the first patient comprises analyzing the first patient using the probe. Typically, it is additionally or alternatively possible that disposing the first patient can include lowering the first patient into an opening within the magnetic resonance imaging system.

Note that not all of the activities described above in the general description or the examples are required, that a portion of a specific activity may not be required, and that one or more further activities may be performed in addition to those described. Still further, the order in which activities are listed is not necessarily the order in which they are performed.

The illustrations of the embodiments described herein are intended to provide a general understanding of the structure of the various embodiments. The illustrations are not intended to serve as a complete description of all of the elements and features of apparatus and systems that utilize the structures or methods described herein. Many other embodiments may be apparent to those of skill in the art upon reviewing the disclosure. Other embodiments may be utilized and derived from the disclosure, such that a structural substitution, logical substitution, or another change may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

The Abstract of the Disclosure is provided to comply with 37 C.F.R. §1.72(b) and is submitted with the understanding that it will not be used to interpret or limit the scope or meaning of the claims. In addition, in the foregoing Detailed Description, various features may be grouped together or described in a single embodiment for the purpose of streamlining the disclosure. This disclosure is not to be interpreted as reflecting an intention that the claimed subject matter requires more features than are expressly recited in each claim. Rather, as the following claims reflect, inventive subject matter may be directed to less than all of the features of any of the disclosed embodiments. Thus, the following claims are incorporated into the Detailed Description, with each claim standing on its own as defining separately claimed subject matter.

Benefits, other advantages, and solutions to problems have been described above with regard to specific embodiments. However, the benefits, advantages, solutions to problems, and any feature(s) that may cause any benefit, advantage, or solution to occur or become more pronounced are not to be construed as a critical, required, or essential feature of any or all the claims.

It is to be appreciated that certain features are, for clarity, described herein in the context of separate embodiments, may also be provided in combination in a single embodiment. Conversely, various features that are, for brevity, described in the context of a single embodiment, may also be provided separately or in any subcombination. Further, reference to values stated in ranges includes each and every value within that range.

The above-disclosed subject matter is to be considered illustrative, and not restrictive, and the appended claims are intended to cover any and all such modifications, enhancements, and other embodiments that fall within the scope of the present invention. Thus, to the maximum extent allowed by law, the scope of the present invention is to be determined by the broadest permissible interpretation of the following claims and their equivalents, and shall not be restricted or limited by the foregoing detailed description.

## Claims

1. A seat (140; 400; 600; 700; 800; 1000; 1200; 1400) for use with a magnetic resonance imaging system comprising:
a first protruding portion (402; 602; 702; 802; 1002; 1006; 1202; 1208; 1402); and
a first sensor (622; 722) disposed within the first protruding portion, wherein the first sensor is capable of providing data for use in an imaging process.

2. The seat of claim 1, wherein the first protruding portion is configured to:
separate buttocks near an anus of a patient;
separate legs of the patient;
separate flesh near a vaginal opening of the patient; or
any combination thereof.

3. A magnetic resonance imaging system (100) comprising the seat of claim 1 and an analyzing region, wherein the seat is disposed within the analyzing region.

4. A chair (120; 200; 300) for use with a magnetic resonance imaging system comprising:
a first section (222; 332) configured to support a back of a patient;
a second section (226; 328) configured to receive a first seat (140; 400; 600; 700; 800; 1000; 1200; 1400) that includes a first sensor (622; 722), wherein the first sensor is capable of providing data for use in an imaging process; and
a bend adjacent to the first section and the second section.

5. The chair of claim 4, wherein the second section is also configured to receive a second seat (140; 400; 600; 700; 800; 1000; 1200; 1400) that includes a second sensor (622; 722), wherein:
the second seat has a different design as compared to the first seat; and
the second sensor is capable of providing data for use in an imaging process.

6. The chair of claim 5, wherein:
the first seat is designed primarily for a male patient as compared to a female patient; and
the second seat is designed primarily for the female patient as compared to the male patient.

7. The chair of any of claims 5 to 6, wherein:
the first seat is designed primarily to analyze a first portion of the patient;
the second seat is designed primarily to analyze a second portion of the patient; the first portion of the patient includes a first organ and not a second organ; and
the second portion of the patient includes the second organ and not the first organ.

8. A method of using a magnetic resonance imaging system (100) comprising:
disposing a first patient within the magnetic resonance imaging system; and
analyzing the first patient using the magnetic resonance imaging system, wherein:
analyzing is performed when the first patient adjacent to a first seat (140; 400; 600; 700; 800; 1000; 1200; 1400) that includes a first sensor (622; 722); and
analyzing is performed using the first sensor.

9. The method of claim 8, further comprising:
removing the first seat (140; 400; 600; 700; 800; 1000; 1200; 1400) from the magnetic resonance imaging system;
placing the second seat into the magnetic resonance imaging system after removing the first seat;
disposing a second patient within the magnetic resonance imaging system after placing the second seat (140; 400; 600; 700; 800; 1000; 1200; 1400) into the magnetic resonance imaging system; and
analyzing the second patient using the magnetic resonance imaging system, wherein:
analyzing is performed when the second patient adjacent to a second seat that includes a second sensor (622; 722);
analyzing is performed using the second sensor; and
the second seat has a different design as compared to the first seat.

10. The method of any of claims 8 to 9, further comprising inserting a probe (1466) into a cavity of the first patient, wherein analyzing the first patient comprises analyzing the first patient using the probe.
